# EUROPEAN PATENT APPLICATION

(11) **EP 3 092 905 A1**
(43) Date of publication of application: **16.11.2016**
(21) Application number: 13899297.9
(22) Date of filing: 17.12.2013
(51) Int. Cl.: A23L 2/02, A23L 33/00

(54) **FUNCTIONAL GRAPE- BASED DRINK CONTAINING, AS ITS MAIN AGENT,POLYPHENOLS OF INTERMEDIATE SIZES IN A LIQUID MATRIX OF PAIS GRAPE JUICE**

(30) Priority: 13.12.2013 CL 2013003571
(71) Applicant: Universidad De Concepcion, Concepción (CL)
(72) Inventor: ROJAS BORQUEZ, Ninón, Concepción (CL); ALBORNOZ ALARCÓN, Francisco, Concepción (CL); FREER CALDERÓN, Juanita, Concepción (CL)
(74) Representative: Elzaburu S.L.P.
(86) International application number: PCT/CL2013/000091
(87) International publication number: WO 2015/085443

(57) **Abstract**

Grape-based functional beverage with intermediate-sized polyphenols as the main ingredient contained within a liquid matrix of pais grape juice.

The present technology consists of a functional beverage based on the juice of the grape *Vitis vinifera* of the Pais variety, enriched with polyphenols of an intermediate molecular weight extracted from red wine grape varieties. This beverage has a positive biological effect on cardiovascular health due to its anti-inflammatory and hypolipidemic properties and improves nitric oxide-dependent vasodilation.

## Description

### FIELD OF THE INVENTION

This technology is oriented towards the food industry and the health sector, mainly the area of functional foods that are effective in the prevention of cardiovascular diseases.

### STATE OF THE ART

Cardiovascular diseases are the main cause of death and disease in Western societies and are a very significant economic burden for the corresponding health systems. Strategies for preventing this type of disease are therefore considered relevant and of high potential for the global population.

Epidemiological studies in southern France have associated a moderate intake of red wine with a 40% decrease in cardiovascular mortality, in spite of a diet high in saturated fats and elevated tobacco consumption (a phenomenon referred to as the "French paradox"). The biological benefit is mainly attributed to a series of compounds known as "polyphenols." Other epidemiological studies have found that the intake of dietary polyphenols is linked to lower mortality caused by ischemic disease. A number of studies, mainly on animal and cellular models, have demonstrated antioxidant, anti-inflammatory, vasoactive and vasodilatory properties on mesenteric and coronary vessels mediated by nitric oxide. Recently, polyphenols have been associated with systemic anti-inflammatory effects, which would contribute to decreasing the progression of chronic vascular inflammation and preserving endothelial function.

Unfortunately, there is limited information regarding the pharmacology of these compounds in human beings, leading to uncertainty as to whether or not they have truly significant *in vivo* biological effects. The main difficulty is due to the fact that they exist as complex mixes presenting enormous variability and not as free compounds, a significant obstacle for studies on bioavailability and physiological effects. In addition, the most abundant polyphenols in a diet are not necessarily those most active in an organism, as not all polyphenols are absorbed with the same efficacy and some are metabolized extensively in the intestine and liver whereas others are rapidly eliminated and excreted. For example, in the case of grapes the total polyphenol content of red wine is 20 times higher than that of white wine, and grape juice contains approximately half the flavonoid concentration of red wine. It has been estimated that red wines contain on average between 1,200 and 4,000 mg/L of total polyphenols. This variability in concentration, not only of total polyphenols but also of the type of polyphenol in different wines and grapes, makes it difficult to interpret the validity of clinical studies with formulations based on "extracts and/or concentrates of total polyphenols" that are commercialized as nutraceutical formulations.

Regarding similar products, in a recent study entitled "Select flavonoids and whole juice from purple grapes inhibit platelet function and enhance nitric oxide release" by Freedman et al., Circulation, 2001, it was observed that the use of red wine grape juice enables a decrease in plaque aggregation, an increase in the concentration of nitric oxide liberated and a decrease in the production of superoxide. In this study, 20 healthy individuals received 500 mL of juice per day.

The study "Purple grape juice improves endothelial function and reduces the susceptibility of LDL cholesterol to oxidation in patients with coronary artery disease" by Stein et al., Circulation, 1999, reports that 700 mL of red wine grape juice increases flow-mediated vasodilation and reduces LDL cholesterol. This suggests that the flavonoid contained in red wine grapes can prevent cardiovascular events.

The study "An innovative grape juice enriched in polyphenols by microwave-assisted extraction" by Al Bittar et al., Food Chemistry 141 (2013), describes a grape juice enriched with polyphenols, extracted by microwave hydrodiffusion and gravity from the same cake obtained from pressing the grapes. The polyphenols obtained are incorporated without prior processing into the juice from the pressing.

### DESCRIPTION OF THE INVENTION

The present technology consists of a nutraceutical formulation in the form of a juice enriched with polyphenols of intermediate molecular weight. When administered, the said juice has a beneficial biological effect on cardiovascular health and can thus be potentially used as a functional food to prevent cardiovascular diseases. Distinguishing features of the invention are the extractive process involved in its production, the molecular size of the polyphenolic formulation and the doses required to achieve a beneficial biological effect in human beings.

The production process for this nutraceutical product involves techniques aimed at maximizing the content of extractable molecules of high value, unlike traditional industrial processes that mainly seek to maximize juice yield. The polyphenols extracted from the pomace (seeds and skin) of different red wine grape varieties are separated according to molecular size using ultrafiltration in order to obtain polyphenols with a size ranging from 1500 Da to 4500 Da. The fraction of polyphenols thus obtained is subsequently incorporated into a liquid matrix in order to produce the functional beverage.

### Polyphenol extraction

The polyphenol concentrate is extracted from the pomace (seeds and skin) remaining after the pressing of red wine grapes such as Tintorera, Cabernet Sauvignon, Syrah or Carménère. It is important to note, however, that no fermentation should take place during the production process in order to avoid a loss of the properties of the polyphenols due to chemical changes. Pomaces from wine production processes are thus not apt for the extraction of these polyphenols.

The pomace obtained through pneumatic pressing to separate it from the juice is ground and subjected to an extractive process with drink ethanol at 50%. This mixture is then filtered to separate the pomace from the mother solution, and the filtered mother solution is purified through food-contact resins in order to eliminate sugars and polysaccharides. The resulting solution is then concentrated and subjected to ultrafiltration to separate it into two fractions: a fraction with a molecular size equal to or lower than 1500 Da and a fraction with a size between 1500 Da and 4500 Da.

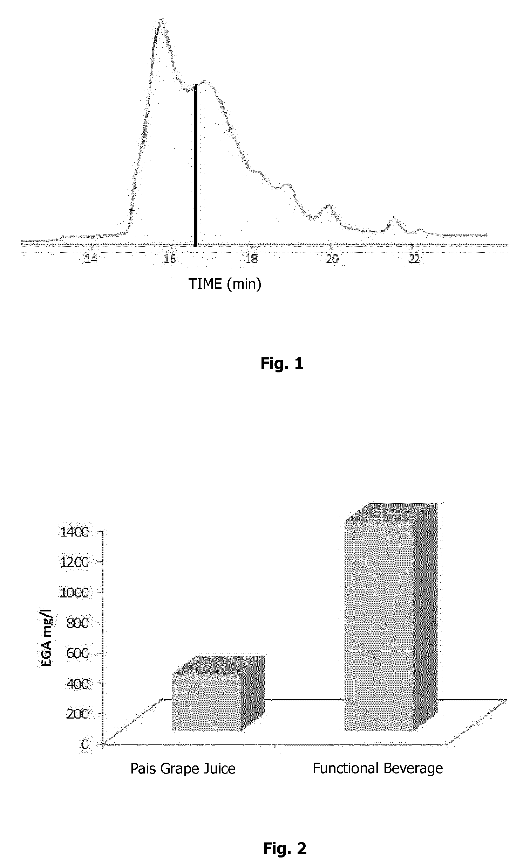

Figure 1 shows the distribution of molecular masses of the polyphenols extracted. The fraction marked with (*) corresponds to the polymeric polyphenols ranging from 1500 Da to 4500 Da, which are the first to appear in the HPLC-GPC column. This separation by columns is merely for analytical purposes, as ultrafiltration is the method used in the production of the functional beverage.

This fractionating makes it possible to exclude small monomers and oligomers smaller than 1500 Da, therefore providing the functional beverage with a higher degree of specificity.

The fraction of polyphenols obtained through separation is then concentrated and spray dried for its subsequent addition to the beverage formulation.

### Functional beverage:

The functional beverage consists of a matrix of grape juice of the Pais variety of *Vitis vinifera* with a polyphenol base concentration of 0,95 mg/L, to which the fraction of larger polyphenols is added in a concentration of 0,1 g/L to 10 g/L, where the preferred concentration is between 1 g/L and 5 g/L. The functional beverage is subsequently poured into individual containers.

The organoleptic characteristics of the beverage were assessed through panel testing. 100% of the judges recognized the samples as grape juice but did not distinguish between grape type and variety. The judges also assessed palatability, describing the beverage as having a light aroma, a slightly astringent taste and a frothy consistency.

In addition, the beverage was analyzed to assess the parameters shown in Table 1.

**Table 1: Characterization of the functional beverage.**

| **Sample** | **Functional beverage A** |
|---|---|
| Soluble solids | 21 °Bx |
| Acidity (pH) | 3,51 |
| Color | Dark ruby |
| Turbidity | 21° |

A measurement of total polyphenols using Folin-Ciocalteu reagent made it possible to determine that the functional beverage has 3.66 times more polyphenols than standard Pais grape juice (see Figure 2).

The functional beverage has a significantly higher antioxidant capacity than Pais grape juice, which has a Trolox equivalent antioxidant capacity (TEAC) of 0.082 mmol/L compared to 0.576 mmol/L in the case of the functional beverage. The functional beverage thus has an antioxidant capacity 7 times greater.

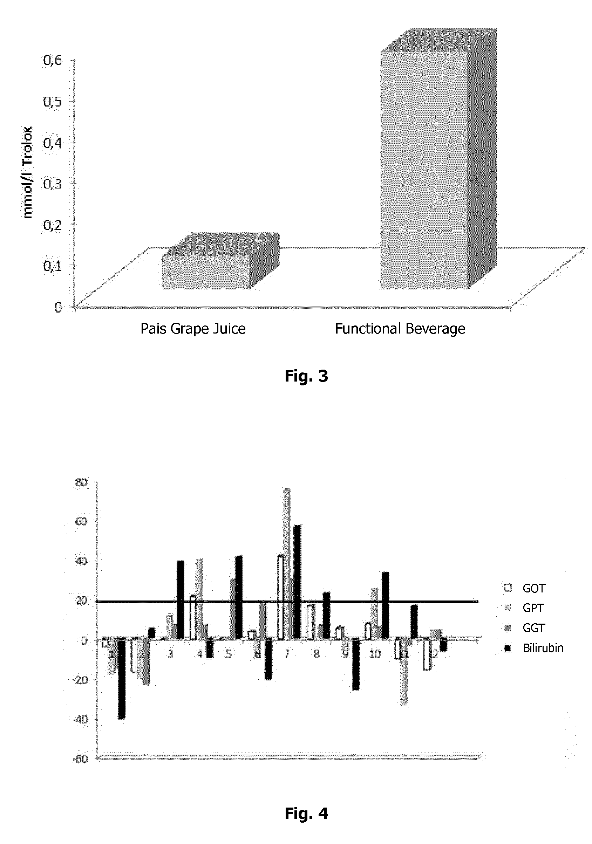

Figure 3 illustrates these data and shows the relationship between polyphenol content and antioxidant capacity. As seen in the figure, a higher polyphenol concentration is associated with a higher antioxidant capacity.

### Clinical assessment

The beverage's properties with regard to the following clinically validated biomarkers associated with atherosclerosis and endothelial dysfunction were evaluated *in vivo:*
a) Endothelial dysfunction, which was clinically assessed based on vasodilatory capacity induced by hyperemic flow. The loss of vasodilatory capacity in the face of these stimuli, as measured in the humeral artery, is an independent predictor of cardiovascular risk.
b) Inflammation, which characterizes all phases of atherothrombotic phenomena and is considered the physiopathological link between the formation of atherosclerotic plaque and its rupture leading to infarctions and arterial occlusion. C-reactive protein (CRP) is the best-characterized inflammation biomarker and is a recognized indicator of cardiovascular risk.
c) Lipid profile, which is considered one of the tools that assist in the diagnosis of cardiovascular diseases, there being a close relation between an increase in cholesterol levels and an elevated risk of coronary cardiovascular diseases (WHO 2011).

Based on clinical studies conducted to evaluate these parameters, it is possible to conclude that the functional beverage has a statistically significant anti-inflammatory, hypolipidemic and nitric oxide-dependent vasodilatory effect. This effect is observed after 4 weeks of use and is maintained until 4 weeks after the last administration of the product. These beneficial physiological effects are not associated with significant adverse events and tolerance to the product was 100%.

In epidemiological terms, the potential applications of this product in cardiovascular prevention are extremely significant in the light of the global obesity and cardiovascular disease pandemic.

### APPLICATION EXAMPLES

### Example 1: Functional Beverage:

### Obtainment of Pais grape juice:

The grapes of the variety Pais were received in 500 kg containers. A visual analysis of the fruit was carried out and the grapes were separated from the stems mechanically using a destemmer. The separated grapes were pressed in a screw press in order to extract the juice and separate the seeds and skin, which were discarded, leaving only the juice. This juice had a total base concentration of polyphenols of 0.95 mg/L. The juice was then filtered to eliminate any remains of fiber or skin. Subsequently, the juice was pasteurized at 60 °C for 30 minutes and refrigerated at 4 °C for two days to carry out the stability analysis.

### Addition of the fractions of polyphenols:

A beverage was produced that contained the fraction of polyphenols of high molecular weight (1500 Da to 4500 Da).

To do this, the juice was heated to 60 °C and the fraction of polyphenols was added at a concentration of 1 g/L, thus enabling individual containers of 250 mL of juice with 250 mg of the active ingredient. The juice was bottled using hot filling in individual glass bottles. After five days, microbiological analysis was carried out and the juice was labeled and packed for dispatch.

### Assessment of total polyphenols using Folin-Ciocalteu reagent

Total polyphenols were measured using Folin-Ciocalteu reagent for the Pais grape juice and the functional beverage containing the polyphenols of high molecular weight. The results obtained are shown in Table 2 and are expressed as mg/L gallic acid equivalents (GAE).

**Table 2: Total Polyphenol Content.**

| **Samples** | **Gallic acid equivalents (mg/l)** |
|---|---|
| Pais grape juice | 376,0 ± 0,1 |
| Functional beverage | 1377,9 ± 0,6 |

| | |
|---|---|
| SD: ± standard deviation | |

### Microbiological analyses

A microbiological analysis of the content of the functional beverage was carried out in accordance with the "Manual of microbiological techniques for food and water 1998" ("Manual de técnicas microbiológicas para alimentos y aguas 1998"), issued by the Public Health Institute of Chile.

### Table 3 contains the results of this analysis.

**Table 3: Microbiological Analysis.**

| **Sample** | **Functional beverage** |
|---|---|
| Aerobic mesophilic count (CFU/g) | 2,6 x 10² |
| *Escherichia coli* (CFU/g) | < 10 |
| Enterobacteria (CFU/g) | < 10 |
| *Staphylococcus aureus* (CFU/g) | < 10 |
| Molds (CFU/g) | < 10 |
| Yeasts (CFU/g) | < 10 |
| *Salmonella spp.* (25 g sample) | absent |

### Example 2: Clinical trials:

Two trial protocols were carried out. The first protocol was a tolerant study of the nutraceutical product in order to obtain the maximum tolerated dose (MTD). The second protocol was a study of the effect of the nutraceutical product on endothelial function. Both trials were carried out with the authorization of the ethics committee of the institution and in accordance with good clinical practice (GCP) standards and Chile's regulatory framework.

### Protocol I: Protocol of Tolerance to the Polyphenol Food Supplement

The study was carried out in the Integrated Cardiovascular Medicine Unit (UAT) at Las Higueras Hospital in Talcahuano, Chile.

The assessment of the maximum tolerated dose for the functional beverage was made using four groups of three voluntary patients, with different dosage schemes. The participants were all healthy volunteers who were not using any type of drugs in accordance with the exclusion and inclusion criteria shown in Table 4.

**Table 4: Inclusion and exclusion criteria of the protocol.**

| **Inclusion criteria** | **Exclusion criteria** |
|---|---|
| Age > 18 | Age < 18 |
| No psychiatric or neurological diseases | Psychiatric or neurological diseases |
| No general diseases | Chronic diseases or regular intake of any medications |
| Normal blood (biochemical) and rest-electrocardiogram tests | Abnormal blood (biochemical) and rest-electrocardiogram tests |
| | Incapacity to provide informed consent |
| | Any condition that according to the judgment of the research team could affect the correct participation of the subject in the trial |

The patients that qualified for the trial were subjected to two days of tests. During the first day, the volunteers, in fasting condition, were subjected to laboratory tests: biochemical profile, urine exam, plasma electrolytes and rest-electrocardiogram. Subjects with clinically significant alterations in their test results were excluded from further participation. The subjects that qualified were given individual bottles of 250 mL of the product containing 500 mg of polyphenols to be ingested orally every day for seven days according to the following scheme:
- Group 1: 500 mg of polyphenols diluted in 250 mL of juice to be ingested at 9 a.m.
- Group 2: 1,000 mg per day divided into one intake of 500 mg in 250 mL of juice at 9 a.m. and another intake of 500 mg in 250 mL of juice at 9 p.m.
- Group 3: 1,500 mg per day divided into 500 mg in 250 mL of juice at 9 a.m., 500 mg in 350 mL of juice at 1 p.m. and 500 mg in 350 mL of juice at 9 p.m.
- Group 4: 2,000 mg per day divided into 500 mg in 250 mL of juice at 9 a.m., 1 p.m., 5 p.m. and 9 p.m. as a daily dose.

Subjects were given a form with dietary instructions in order to control the intake of dietary polyphenols.

After seven days of product intake, the subjects, in fasting condition, were subjected to the second day of tests, where the tolerance questionnaire, a physical exam, and biochemical, urine and electrocardiogram tests were carried out.

As part of the tests, the volunteers completed a questionnaire to ascertain their tolerance of the food supplement containing polyphenols *("Encuesta de Tolerancia Suplemento alimentario polifenoles")* Table 5 contains the symptoms described by the volunteers.

**Table 5: Total symptoms and signs of the study participants.**

| | **Volunteers** | **N° of symptoms** |
|---|---|---|
| **Group 1** | **J001** | 0 |
| | **J002** | 1 |
| | **J003** | 0 |
| **Group 2** | **J004** | 6 |
| | **J005** | 6 |
| | **J006** | 1 |
| **Group 3** | **J007** | 2 |
| | **J008** | 2 |
| | **J009** | 1 |
| **Group 4** | **J010** | 2 |
| | **J011** | 1 |
| | **J012** | 7 |
| | **J013** | 2 |

The results show that of the four groups, the most symptoms were reported by participants from Group 4 and Group 2. The volunteers from Group 1 reported the least number of effects and had the best tolerance to the beverage. It is important to note that there was no linear relationship between the daily dose and the number of symptoms.

If we individualize the most common recurring symptoms, the most reported symptom from volunteers was an "increase in intestinal transit" (54% of volunteers). Some volunteers who had habitually suffered from constipation refer to this symptom as beneficial. The second most reported symptom was a lack of appetite, weight change and nausea (23%). In third place was epigastralgia, abdominal pain and anxiety (15%). The remaining symptoms, corresponding to 7,5%, were headaches, vertigo, precordial pain, vomiting, liquid stools, thirst, polyuria, and muscle pain.

**Table 6: Signs and Symptoms in Volunteers:**

| **Sign or symptom** | **Amount** |
|---|---|
| Lack of appetite | 3 |
| Weight change | 3 |
| Headaches | 1 |
| Vertigo | 1 |
| Precordial pain | 1 |
| Cardiovascular pain | 0 |
| Respiratory pain | 0 |
| Epigastralgia | 2 |
| Abdominal pain | 2 |
| Nausea | 3 |
| Vomiting | 1 |
| Diarrhea | 1 |
| Skin | 0 |
| Fluids/electrolytes (thirst) | 1 |
| Renal/urinary (polyuria/dysuria) | 1 |
| Hematopoietic | 0 |
| Muscle pain | 1 |
| Anxiety, nervousness | 2 |
| Gastrointestinal (increased transit) | 7 |

Toxicity was considered as an increase to more than twice the maximum normal levels of hepatic transaminases, alkaline phosphatases, and total and conjugated bilirubin; an increase of more than 20% over baseline in liver values or blood creatinine, or a decrease in the creatinine clearance calculated; an increase in glycemia to diagnostic ranges for type II diabetes; and a prolongation of Qtc higher than 20% of the baseline value. This is obtained by comparing the results from study day 1 to those of study day 2. Nausea, vomiting, diarrhea, abdominal pain, pyrosis, and flatulence were considered adverse gastrointestinal effects.

The maximum tolerated dose (MTD) was calculated in the following way: Three volunteers were administered a certain dose level. If toxicity was not observed in any of the volunteers, the dose was increased to the next level. If toxicity was observed in at least one individual per group, the same dose level was repeated in another group. If toxicity was observed again, the previous dose was declared the MTD.

Data analysis made it possible to establish that the formulation with 500 mg was associated with hepatotoxicity when three bottles of the product per day were ingested. Hepatotoxicity was defined as an increase of more than 20% compared to base levels in gamma-glutamyl transferase (GGT), serum glutamic-oxaloacetic transaminase (SGOT), serum glutamic pyruvic transaminase (SGPT) and blood bilirubin. This can be seen in Figure 4, which shows the percentage of the difference between the values obtained on days 1 and 2, with a toxicity increase of 20% as a reference.

When analyzing other parameters, it was observed that the same dose did not affect alkaline phosphatases or other biochemical parameters. No changes in glucose metabolism or electrocardiogram alterations were observed.

Table 7 shows the glycemia values, where toxicity was defined as a glycemic increase to diagnostic ranges for type II diabetes, i.e. equal to or greater than 110 mg/dL in fasting condition.

**Table 7: Glycemia Values for Healthy Volunteers.**

| | **Day 1** | **Day 2** |
|---|---|---|
| **J001** | 91 | 89 |
| **J002** | 77 | 80 |
| **J003** | 85 | 93 |
| **J004** | 94 | 82 |
| **J005** | 90 | 89 |
| **J006** | 97 | **103** |
| **J007** | 83 | 77 |
| **J008** | 84 | 71 |
| **J009** | 75 | 88 |
| **J010** | 85 | 90 |
| **J011** | 84 | 80 |
| **J012** | 90 | **106** |
| **J013** | 75 | 77 |

All patients had a normal uremia, blood urea nitrogen (BUN), alkaline phosphatase (ALP) and blood creatinine levels on days 1 and 2. On days 2, uremia and BUN values increased above base level but did not reach the normal maximum. Likewise, only one patient in group 3 showed an elevation of more than 20% in ALP compared to base, but this increase did not occur in the next dose group.

Based on these results, the results of the laboratory tests, the tolerances described, and the safe intake levels, a juice product was formulated with 250 mg of polyphenols per bottle to be used with the following protocols. In addition, a maximum daily intake of 250 mg of polyphenols was recommended.

### Protocol II: Study of the effect of the functional beverage on endothelial function.

In this trial the functional beverage was assessed: 250 mL of juice with 250 mg of polyphenols of intermediate molecular weight (1500 Da - 4000 Da) extracted from red wine grapes, taken once daily, by volunteers with cardiovascular risk factors.

### Methodology.

The trial was a double-blind (patient and researcher), randomized and crossover trial lasting 10 weeks. The design allowed each volunteer to be his or her own control, decreasing the size of the sample required to demonstrate the physiological effect sought and to reduce selection and observation bias.

Individuals fulfilling the inclusion/exclusion criteria (see Table 8) were invited to participate voluntarily in the study. After obtaining informed consent, 70 subjects were screened, of which 35 were selected because they fulfilled the inclusion/exclusion criteria and had high-sensitivity C-reactive protein (ₕₛPCR) levels equal to or greater than 2 mg/dL. From these, 18 subjects were assigned to a placebo (Group A) and 17 to an active product (Group B).

**Table 8: Inclusion and Exclusion Criteria.**

| **Inclusion criteria** | **Exclusion criteria** |
|---|---|
| 1. Men and women > 18 years of age | 1. Refusal to sign informed consent. |
| 2. At least 2 cardiovascular risk factors such as: | 2. Acute or chronic infections at the time of inclusion. |
| a) overweight/obesity, | 3. Acute or chronic inflammatory disease. |
| b) dyslipidemia, DM2, | 4. Acute or chronic liver disease. |
| c) tobacco consumption, | 5. Alcohol or drug addiction. |
| d) sedentary lifestyle, | 6. Neoplastic disease. |
| e) coronary heart disease, | 7. Decompensated cardiac insufficiency. |
| f) history of stroke or peripheral vascular disease, | 8. New user of statins. |
| | 9. Anti-inflammatory or immunosuppressant treatment. |
| g) family history of coronary diseases, | |
| h) hsPCR greater than 2 mg/dL, | 10. History of hypersensitivity to the trial product. |
| i) age above 55 in men and above 55 and women. | |
| | 11. Pregnancy. |
| | 12. Simultaneous participation in another clinical or pharmacological research trial. |
| | 13. Any other condition that the research team considers inappropriate for participation in the study. |

Each patient was subjected to four days of trial, in which the biochemical and physiological measurements were carried out and subjects were briefed and given the trial product. These study days were carried out at the beginning (base or study day 1), after four weeks of treatment (study day 2), after a period of suspension of the treatment (washout period) lasting two weeks (study day 3), and at the end of the study after four weeks of changed treatment (study day 4). Table 9 shows the design of the study.

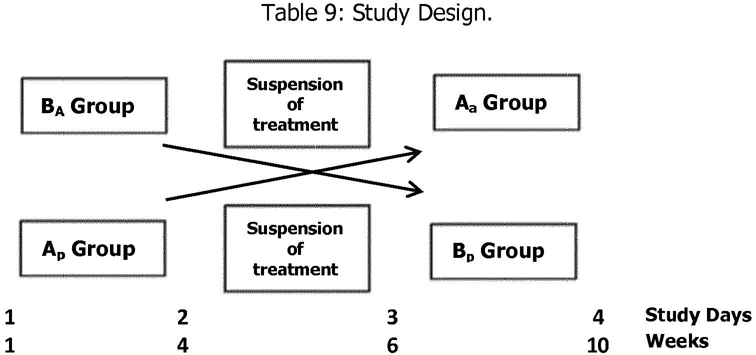

On study days, the subjects were subjected to a clinical history, general physical checkup, and sampling to carry out the following tests:
a) General biochemical tests: blood count, biochemical profile, complete urine, lipid profile, and electrocardiogram.
b) hsPCR: high-sensitivity C-reactive protein.
c) FMD: brachial arterial flow-mediated dilation, via ultrasound.

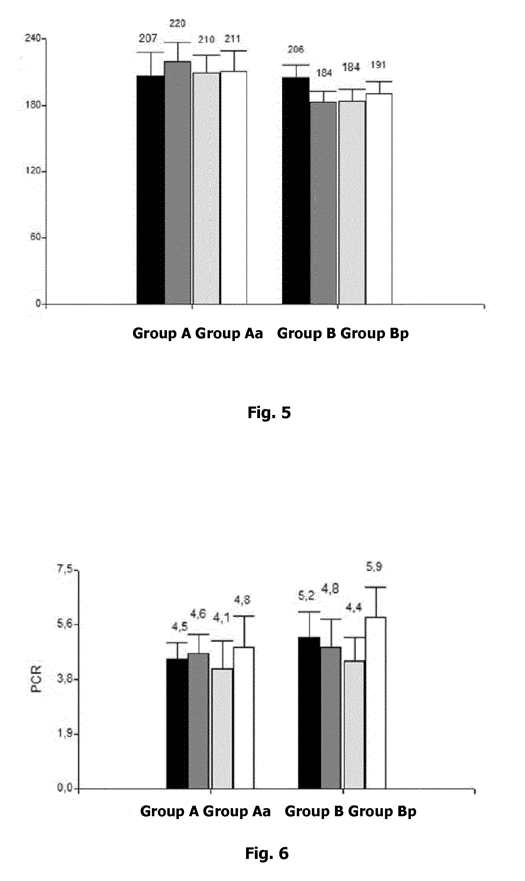

For the general biochemical analysis, a sample of venous blood was taken using a tube with clot activator. Plasma was separated by centrifuging the total blood at 3,000 rpm for three minutes and was analyzed immediately after being obtained at the main laboratory of Las Higueras Hospital. Among the results obtained, it was possible to observe a significant decrease in total plasma cholesterol levels compared to placebo (see Figure 5), which demonstrates the product's hypolipidemic effect.

For hsPCR measurements, a venous blood sample was taken using a tube with clot activator. Plasma was separated by centrifuge at 3,000 rpm for three minutes and the sample was frozen at -70 °C for its subsequent nephelometric analysis at the main laboratory of Las Higueras Hospital. The results obtained are shown in Figure 6. It is possible to observe that individuals that ingested the active product show a significant decrease in plasma hsPCR levels that lasts up to four weeks after treatment suspension. This demonstrates the product's anti-inflammatory effect.

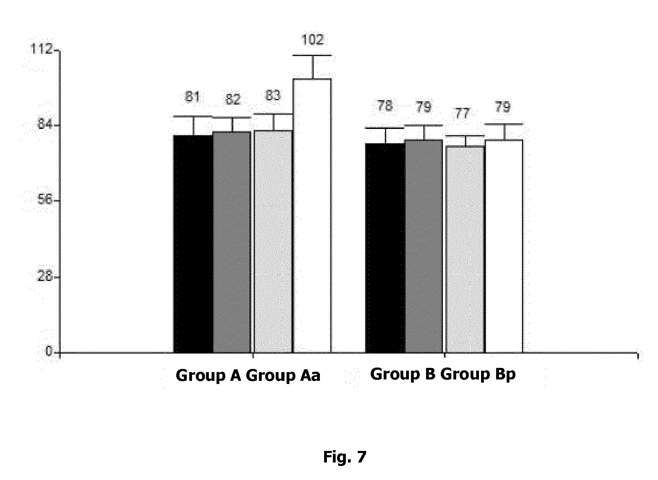

To measure vasodilatory capacity using ultrasound, a 2D echocardiography was carried out with color and spectral Doppler and vascular ultrasound from 7 MHz to 12 MHz with continuous ECG monitoring and an adequate tensiometer cuff for the patient's arm. Volunteers were instructed not to smoke, exercise, or consume caffeine in the previous 4-6 hours, and not to eat fat or foods with vitamin C at least 12 hours prior to the tests on the study day. Subjects were given an appointment on the study day and instructed to rest in supine decubitus position for at least 10 minutes at room temperature. The brachial artery was imaged above the antecubital fossa in a longitudinal plane. The image was obtained in grayscale to clearly distinguish the lumen of the brachial artery (humeral) and the anterior and posterior intima-media complex. Then a cuff typically used to measure arterial tension was placed on the subject's arm. The diameter of the brachial arteria was measured along the long axis in 2D and M mode. After the basal diameter was obtained, the cuff was inflated to a minimum of 20 mmHg above the subject's systolic pressure, monitoring arterial flow on the monitor of the ultrasound machine, to produce a state of ischemia for 5 minutes. The cuff was subsequently deflated, restoring flow to the brachial artery and producing hyperemia. After one minute, the diameter of the brachial artery was measured once again. A normal response (normal endothelial function) is an increase of at least 10% in diameter compared to base measurement. Basal conditions return completely after approximately 10 minutes of rest. The variables measured included absolute diameter of the artery, from intima to intima in basal conditions, one minute after cuff deflation, and the percentage of change after ischemia. The comparison of post-ischemic systolic velocity after one minute is shown in Figure 7, where it is possible to observe a significant increase in the velocity of the group that received the active product compared to the placebo. This demonstrates an improvement in vasodilatory capacity in the volunteers subjected to this treatment.

To analyze biomarker data, Wilcoxon Signed rank test was used for paired samples with non-normal distribution and *p* ≤ 0.05. Table 10 shows the results of these analyses. Values are expressed as means with standard deviation. The units of measurement were the same for both groups.

**Table 10: Summary of Biomarker Values.**

| **Group A** | **Study day 1** | **Study day 2** | **Study day 3** | **Study day 4** |
|---|---|---|---|---|
| hsPCR (mg/dL) | 4,47 + 1,76 | 4,62 + 2,12 | 4,14 + 2,98 | 4,83 + 3,53 |
| Total cholesterol (mg/dL) | 206,50 + 68,11 | 220,00 + 55,10 | 209,50 + 50,63 | 211,00 + 58,65 |
| Average weight (kg) | 75,00 + 12,10 | 76,70 + 12,53 | 76,30 + 12,60 | 77,00 + 12,55 |
| QTc (milliseconds) | 428,00 + 34,70 | 412,00 + 25,07 | 431,00 + 26,41 | 419,00 + 35,77 |
| Post-ischemic systolic velocity after 1 min (cm/second) | 80,50 + 24,08* | 82,00 + 17,78 | 82,50 + 19,52*** | 102,00 + 26,51**** |
| Immediate systolic velocity | 104,00 + 24,84 | 100,50 + 23,82 | 114,10 + 31,47 | 143,00 + 27,42 |
| Immediate post-ischemic humeral artery diameter | 0,40 + 0,09 | 0,42 + 0,04 | 0,41 + 0,05 | 0,43 + 0,04 |
| Post-ischemic humeral artery diameter after 1 min (cm) | 0, 39 + 0,11 | 0,42 + 0,05 | 0,43 + 0,07 | 0,44 + 0,04 |
| SGOT (mg/dL) | 19,50 + 10,15 | 22,00 + 9,13 | 19,50 + 5,92 | 19,00 + 8,01 |
| SGPT (mg/dL) | 22,50 + 16,89 | 27,00 + 17,42 | 23,50 + 10,09 | 22,00 + 13,39 |
| GGT (mg/dL) | 32,00 + 25,34 | 35,00 + 27,70 | 32,50 + 38,26 | 30,00 + 26,25 |

| **Group B** | **Study day 1** | **Study day 2** | **Study day 3** | **Study day 4** |
|---|---|---|---|---|
| hsPCR | *5,20 + 2,82 | **4,84 + 3,13 | ***4,38 + 2,57 | 5,87 + 3,30 |
| Total cholesterol | 205,50 + 35,43 | 183,50 + 30,76 | 184,00 + 33,32 | 190,50 + 37,53 |
| Average weight | 78,40 + 19,77 | 78,75 + 19,36 | 78,10 + 19,05 | 77,30 + 19,47 |
| QTc | 423,00 + 24,19 | 420,00 + 22,42 | 426,50 + 20,37 | 419,00 + 21,88 |
| Post-ischemic systolic velocity after 1 min | 77,82 + 18,71 | 79,00 + 17,24 | 76,50 + 12,59 | 79,00 + 18,23 |
| Immediate systolic velocity | 101,38 + 23,51 | 103,50 + 25,92 | 108,00 + 27,47 | 119,00 + 38,02 |
| Immediate post-ischemic humeral artery diameter | 0,42 + 0,07 | 0,44 + 0,06 | 0,43 + 0,09 | 0,46 + 0,07 |
| Post-ischemic humeral artery diameter after 1 min | 0,45 + 0,07 | 0,41 + 0,07 | 0,41 + 0,08 | 0,44 + 0,07 |
| SGOT | 23,00 + 12,88 | 23,00 + 17,09 | 25,00 + 13,74 | 23,50 + 15,43 |
| SGPT | 29,00 + 30,81 | 30,50 + 35,49 | 29,50 + 36,41 | 32,50 + 30,09 |
| GGT | 23,00 + 27,28 | 23,00 + 32,29 | 22,00 + 30,42 | 22,00 + 28,92 |

## Claims

1. A functional, grape-based beverage **CHARACTERIZED in that** it contains as the main ingredient added polyphenols obtained from red wine grapes, which are fractionated by size, within a liquid matrix of Pais grape juice.

2. A functional, grape-based beverage **CHARACTERIZED in that** the added polyphenols exhibit a size between 1500 Da and 4500 Da.

3. A functional, grape-based beverage, according to claim 1, **CHARACTERIZED in that** the added polyphenols are obtained from the pomace of red wine grapes such as Tintorera, Cabernet Sauvignon, Syrah or Carménère, used in juice production.

4. A functional, grape-based beverage, according to claim 1, **CHARACTERIZED in that** the polyphenols have a concentration of 0.1 g/L to 10 g/L.

5. A functional, grape-based beverage, according to claim 1, **CHARACTERIZED in that** it has a light aroma, a slightly astringent taste, and a frothy consistency.

6. A functional, grape-based beverage, according to claim 1, **CHARACTERIZED in that** it has a concentration of soluble solids of 21 °Bx, a pH of 3.51, and a turbidity of 21°.

7. A functional, grape-based beverage, according to claim 1, **CHARACTERIZED in that** it has 3.66 times more polyphenols than Pais grape juice.

8. A functional, grape-based beverage, according to claim 1, **CHARACTERIZED in that** it has an antioxidant capacity 7 times greater than Pais grape juice.

9. A functional, grape-based beverage, according to claim 1, **CHARACTERIZED in that** it has an anti-inflammatory and hypolipidemic effect.

10. A functional, grape-based beverage, according to claim 1, **CHARACTERIZED in that** it enables an improvement in nitric oxide-dependent vasodilatory capacity.

## Amended claims

### Amended claims under Art. 19.1 PCT

**1.** A functional, grape-based beverage **CHARACTERIZED in that** it contains as the main ingredient added polyphenols obtained from red wine grapes with a size between 1500 Da and 4500 Da within a liquid matrix of Pais grape juice.

**2.** A functional, grape-based beverage, according to claim 1, **CHARACTERIZED in that** the added polyphenols are obtained from the pomace of red wine grapes such as Tintorera, Cabernet Sauvignon, Syrah or Carménère, used in juice production.

**3.** A functional, grape-based beverage, according to claim 1, **CHARACTERIZED in that** the polyphenols have a concentration of 0.1 g/L to 10 g/L.

**4.** A functional, grape-based beverage, according to claim 1, **CHARACTERIZED in that** it has a light aroma, a slightly astringent taste, and a frothy consistency.

**5.** A functional, grape-based beverage, according to claim 1, **CHARACTERIZED in that** it has a concentration of soluble solids of 21 °Bx, a pH of 3.51, and a turbidity of 21°.

**6.** A functional, grape-based beverage, according to claim 1, **CHARACTERIZED in that** it has 3.66 times more polyphenols than Pais grape juice.

**7.** A functional, grape-based beverage, according to claim 1, **CHARACTERIZED in that** it has an antioxidant capacity 7 times greater than Pais grape juice.

**8.** A functional, grape-based beverage, according to claim 1, **CHARACTERIZED in that** it has an anti-inflammatory and hypolipidemic effect.

**9.** A functional, grape-based beverage, according to claim 1, **CHARACTERIZED in that** it enables an improvement in nitric oxide-dependent vasodilatory capacity.
